# EUROPEAN PATENT APPLICATION

(11) **EP 0 675 194 A1**
(43) Date of publication of application: **04.10.1995**
(21) Application number: 94870055.4
(22) Date of filing: 28.03.1994
(51) Int. Cl.: C11D 17/00

(54) **Detergent additives in structured liquids**

(71) Applicant: THE PROCTER & GAMBLE COMPANY, Cincinnati, Ohio 45202 (US)
(72) Inventor: Cauwberghs, Serge Gabriel Pierre Roger, B-9100 Nieuwkerken (BE); Lappas, Dimitris (NMN), B-1020 Brussels (BE)
(74) Representative: Canonici, Jean-Jacques

(57) **Abstract**

The present invention relates to structured liquid detergents comprising porous hydrophobic materials, the porous hydrophobic materials having a detergent active absorbed therein, said hydrophobic material subsequently being coated with hydrophobic material.

## Description

### Field of the invention

The present invention relates to detergent additives in structured liquid detergent compositions.
More in particular, the present invention relates to structured liquid detergents comprising porous hydrophobic materials, the porous hydrophobic materials having a detergent active absorbed therein, said hydrophobic material subsequently being coated with hydrophobic material.

### Background of the invention

Structured liquid detergents have been extensively described in the art, with a view to suspend solid particles which are insoluble in the detergent matrix. In non-structured liquid detergents, the presence of such ingredients generally leads to sedimentation or phase separation and therefore renders such detergents unacceptable from a consumer's viewpoint.

In response to this need, detergent formulators have designed structured liquid detergents which have the capability of stably suspending solids.

Such structured liquids can be "internally structured", whereby the structure is formed by primary ingredients, and/or they can be structured by secondary additives which can be added to a composition as "external structurants". Both forms of structuring are well known in the art. External structuring is usually used for the purpose of suspending solid particles and/or droplets of nonionic surfactant. Internal structuring is usually used to suspend particles and/or to endow properties such as consumer-preferred flow properties and/or turbid appearance. Examples of internally structured liquid and externally structured liquid detergent compositions are given in EP-A-414 285.

However, it is equally recognized that the relative ability of the structured liquid to be effective in suspending solid particles is among others depending on the nature and concentration of the suspended particle. Upon storage, said suspended particles have a tendency to sediment resulting in liquid detergents which are less effective, having poor appearance and which present physical stability issues. Examples of such suspended particles are detergency builders and abrasive particles.

Surprisingly, it has now been found that porous hydrophobic particles having a detergent active absorbed therein, said hydrophobic material being coated with hydrophobic material are very suitable for incorporation in structured liquids.
In addition, it has been found that the hydrophobic material of the present invention can be easily incorporated into the liquid detergent matrix without the need of an emulsifier or excessive mechanical agitation.

Said hydrophobic particles can serve to protect detergent active compounds by isolating and protecting said compounds from their hostile environment upon storage but releasing said compounds during washing conditions. Examples of said compounds include perfumes, enzymes, bleaches and catalysts.

Accordingly, this finding allows us to formulate stable liquid detergent compositions containing protected detergent active compounds.

Furthermore, it has been found that the porous hydrophobic materials when incorporated in structured liquids, are more efficient in protecting the detergent active compounds.
EP 583 512 describes the use of porous hydrophobic materials suitable for protection of detergent active compounds.

### Summary of the invention

The present invention relates to structured liquid detergents comprising porous hydrophobic materials, the porous hydrophobic materials having a detergent active absorbed therein, said hydrophobic material subsequently being coated with hydrophobic material.

### Detailed description of the invention

### Structured liquid detergent composition

The compositions according to the present invention are structured liquid detergent compositions which can be either internally or externally structured. Structured liquid detergent compositions are described in EP 414 549, EP 295 021 and EP 346 994.

Preferred structured liquid detergent compositions suitable for the present invention include detergent compositions formulated with anionic surfactants and hydrophilic polyhydroxy fatty acid amide surfactant such as described in EP 572 723. Preferred polyhydroxy fatty acid amide surfactant component for use in the present invention comprises compounds of the structural formula : 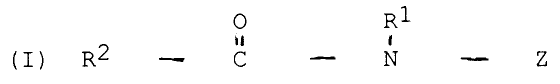
wherein : R¹ is H, C1-C4 hydrocarbyl, 2-hydroxy ethyl, 2-hydroxy propyl, or a mixture thereof, preferably C₁-C₄ alkyl, more preferably C₁ or C₂ alkyl, most preferably C₁ alkyl (i.e. methyl); and R² is a C₅-C₃₁ hydrocarbyl, preferably straight chain C₇-C₁₉ alkyl or alkenyl, more preferably straight chain C₉-C₁₇ alkyl or alkenyl, most preferably straight chain C₁₁-C₁₇ alkyl or alkenyl, or mixtures thereof; and Z is a polyhydroxyhydrocarbyl having a linear hydrocarbyl chain with at least 3 hydroxyls directly connected to the chain, or an alkoxylated derivative (preferably ethoxylated or propoxylated) thereof. Z preferably will be derived from a reducing sugar in reductive amination reaction; more preferably Z is a glycityl. Suitable reducing sugars include glucose, fructose, maltose, lactose, galactose, mannose and xylose. As raw materials, high dextrose corn syrup, high fructose corn syrup, and high maltose corn syrup can be utilized as well as the individual sugars listed above. It should be understood that these corn syrups may yield a mix of sugar components for Z. Z preferably will be selected from the group consisting of -CH₂-(CHOH)ₙ-CH₂OH, -CH(CH₂OH)-(CHOH)ₙ₋₁-CH₂OH, -CH₂-(CHOH)₂-CHOR')-CHOH)-CH₂OH, and alkoxylated derivatives thereof, where n is an integer from 3 to 5, inclusive, and R' is H or a cyclic or aliphatic monosaccharide. Most preferred are glycityls wherein n is 4, particularly -CH₂-(CHOH)₄-CH₂OH.

In formula (I), R¹ can be,for example, N-methyl, N-ethyl, N-propyl, N-isopropyl, N-butyl, N-2-hydroxy ethyl, or N-2-hydroxy propyl.

R²-CO-N< can be for example, cocamide, stearamide, oleamide, lauramide, myristamide, capricamide, palmitamide, tallowamide, etc.

Z can be 1-deoxyglucityl, 2-deoxyfructityl, 1-deoxymaltityl, 1-deoxylactityl, 1-deoxygalactityl, 1-deoxymannityl, 1-deoxymaltotriotityl, etc.

Other preferred structured liquid detergent compositions suitable for the present invention includes externally structured detergent compositions with clay.
Such structured compositions are very suitable for serving as softening-through-the wash liquid detergent compositions., The clay is present in an amount of at least 0.5%, preferably from 1% to 10%, more preferably from 2% to 5% by weight of the detergent composition. The preferred clays are of the smectite type. Smectite type clays are widely used as fabric softening ingredients in detergent compositions. Most of these clays have a cation exchange capacity of at least 50 meq./100g. Smectite clays can be described as three-layer expandable materials, consisting of alumino-silicates or magnesium silicates.

There are two distinct classes of smectite-type clays; in the first, aluminium oxide is present in the silicate crystal lattice, in the second class of smectites, magnesium oxide is present in the silicate crystal lattice. The general formulas of these smectites are A1₂(Si₂0₅)₂(OH)₂ and Mg₃(Si₂O₅) (OH)₂, for the aluminium and magnesium oxide type clay, respectively. The range of the water of hydration can vary with the processing to which the clay has been subjected. Furthermore, atom substitution by iron and magnesium can occur within the crystal lattice of the smectites, while metal cations such as Na⁺, Ca²⁺, as well as H⁺ can be co-present in the water of hydration to provide electrical neutrality. It is customary to distinguish between clays on the basis of one cation predominantly or exclusively absorbed. For example, a sodium clay is one in which the absorbed cation is predominantly sodium. Such absorbed cations can become involved in equilibrium exchange reactions with cations present in aqueous solutions. In such equilibrium reactions, one equivalent weight of solution cation replaces an equivalent of sodium, for example, and it is customary to measure clay cation exchange capacity in terms of milliequivalents per 100g. of clay (meq/100g.). The cation exchange capacity of clays can be measured in several ways, including electrodialysis, by exchange with ammonium ion followed by titration, or by a methylene blue procedure, all as set forth in Grimshaw, The Chemistry and Physics of Clays, Interscience Publishers, Inc. pp. 264-265(1971). The cation exchange capacity of a clay mineral relates to such factors as the expandable properties of the clay, the charge of the clay, which in turn, is determinated at least in part by the lattice structure, and the like. The ion exchange capacity of clays varies widely in the range from about 2 meq/100 g. for kaolinites to about 150 meq/100 g., and greater, for certain clays of the montmorillonite variety. Illite clays have an ionexchange capacity somewhere in the lower portion of the range, ca. 26 meq/100 g. for an average illite clay. It has been determined that illite and kaolinite clays, with their relatively low ion exchange capacities, are not useful in the instant compositions. Indeed such illite and kaolinite clays constitute a major component of clay soils. However, smectites, such as nontronite having an ionexchange capacity of approximately 50 meq/100 g.;saponite, which has an ion exchange capacity greater than 70 meq/100g., have been found to be useful fabric softeners.

The smectite clays commonly used for this purpose herein are all commercially available. Such clays include, for example, montmorillonite, volchonskoite, nontronite, hectorite, saponite, sauconite, and vermiculite. The clays herein are available under commercial names such as "fooler clay" (clay found in a relatively thin vein above the main bentonite or montmorillonite veins in the Black Hills) and various tradenames such as Thixogel #1 (also, "Thixo-Jell") and Gelwhite GP from Georgia Kaolin Co. Elizabeth, New Jersey; Volclay BC and Volclay #325, from American Colloid Co., Skokie, Illinois; Black Hills Bentonite BH 450, from International Minerals and Chemicals; and Veegum Pro and Veegum F, from R.T. Vanderbilt. It is to be recognized that such smectite-type minerals obtained under the foregoing commercial and tradenames can comprise mixtures of the various discrete mineral entitites. Such mixtures of the smectite minerals are suitable for use herein.

Preferred for use herein are the montmorrillonite clays.

Quite suitable are hectorites of natural or synthetic origin, in the form of particles having the general formula 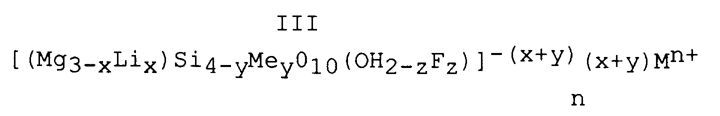
wherein Me^{III} is Al, Fe, or B; or y=o; Mⁿ⁺ is a monovalent (n=1) or divalent (n=2) metal ion, for example selected from Na, K, Mg, Ca, Sr.

In the above formula, the value of (x+y) is the layer charge of the hectorite clay.

Such hectorite clays are preferably selected on the basis of their layer charge properties, i.e. at least 50% is in the range of from 0.23 to 0.31.

More suitable are hectorite clays of natural origin having a layer charge distribution such that at least 65% is in the range of from 0.23 to 0.31.

The hectorite clays suitable in the present composition should preferably be sodium clays, for better softening activity.

Sodium clays are either naturally occuring, or are naturally-occuring calcium-clays which have been treated so as to convert them to sodium-clays. If calcium-clays are used in the present compositions, a salt of sodium can be added to the compositions in order to convert the calcium clay to a sodium clay. Preferably, such a salt is sodium carbonate, typically added at levels of up to 5% of the total amount of clay.

Examples of hectorite clays suitable for the present compositions include Bentone EW and Macaloid, from NL Chemicals, N.J., U.S.A., and hectorites from Industrial Mineral Ventures.
Combinations of clays can be used to improve the stability of the structured liquid. Example of such a combination includes montmorrilonite and hectorite clays (e.g. organoclays).
Typically, the clays are processed in the liquid detergent composition by high pressure homogenisation.

### Porous hydrophobic material

The porous hydrophobic material suitable for the present invention can be any hydrophobic porous material. The average pore diameter of the porous hydrophobic material should be larger than the size of the detergent active compound that is to be absorbed in the porous material. Pore volumes and pore size distributions may be measured by the recognized technique of mercury intrusion porosimetry.

For instance, if the detergent active compound is an enzyme, an average pore diameter of the hydrophobic material of 500 angstroms or higher is preferred.

The preferred hydrophobic material that then can be used is silica. The average pore diameter of the currently used silica is 1000 Å while the absorbed enzyme molecules have diameters in the range of 50 to 150 Å.

The silica particles can be rendered hyrophobic by treating them with dialkylsilane groups and/or trialkylsilane groups either bonded directly onto the silica or by means of silicone resin. The silica is further characterized by a high absorption value. The absorption can be expressed as Dibutylphthalate (DBP) number. Porous silica suitable for the present invention is available under the trade name Sipernat^{(R)} from Degussa.

The porous hydrophobic material is coated with a hydrophobic coating material. The level of hydrophobic coating material should be such that appropriate coverage of all additive-filled porous hydrophobic material is secured.

The hydrophobic coating material is preferably a hydrophobic liquid polymer. Such a polymer may be an organo polysiloxane oil, e.g. a polydi(alkyl) siloxane, especially a polydi(methyl) siloxane. Especially preferred are hydrophobic silicone oil which have been proposed for use as antifoam in liquid detergents.
Alternatively the hydrophobic material may be a high molecular weight hydrocarbon like petroleum jelly, wax, or water insoluble but water permeable materials such as caprolactones.
The hydrophobic coating material provides a protective coating for the active-filled porous hydrophobic materials herein. Coating the pores of the hydrophobic material isolates the detergent active compound from environments which causes the degradation of the active compounds. The active compounds remain in their stabilized material environment without interacting with other potentially harmful detergent ingredients or the environment is protected from the detergent active compound itself. Said hydrophobic materials when incorporated in structured liquids have found to be extremely useful in protecting and delivering a detergent active in terms of storage stability and performance.
In addition, the improved stability of said porous hydrophobic materials in structured liquid detergents allows to increase the amount of detergent active to be protected which, in turn, improves the detergency performance.
Furthermore, the hydrophobic material of the present invention can be easily incorporated into the liquid detergent matrix without the need of an emulsifier or excessive mechanical action.
Typically, the porous hydrophobic material of the present invention will be simply added in the continuous processing of the liquid detergent. In the case the liquid detergent is structured by the clay, then the porous hydrophobic material is preferably added after the high pressure homogenisation process step.

The detergent active compounds suitable for the present invention include enzymes, bleaches, bleach activators. bleach catalysts, perfumes, photoactivators, dyes, brighteners/fluorescers, through the wash sanitizers, fabric softening or conditioning agents, hydrolysable surfactants, polymers and other detergent active compounds which are water-soluble or water-dispersible and mixtures thereof.

A preferred class of a detergent active compound is a detergency enzyme. Examples of enzymes suitable for the present invention are enzymes which are active in the removal of soils or stains such as protease, lipase, amylase, carboxylase, cellulase, oxidase, peroxidase or mixtures thereof.

The enzyme may be present in the form of an enzyme solution, e.g. in water or a lower water miscible, mono-,di- or polyhydric alcohol such as propylene glycol and optionally containing enzyme stabilizers such as is known in the art. Enzyme stabilizers which may be present include lower alcohols, e.g. glycerol, lower mono- or di- carboxylic acid and their salts, especially formates and oxidates, borates and calcium salts. Preferred enzymes are cellulases. Said cellulases include both bacterial or fungal cellulases. Preferably, they will have a pH optimum of between 5 and 9.5. Suitable cellulases are disclosed in U.S. Patent 4,435,307, Barbesgoard et al, issued March 6, 1984, incorporated herein by reference, which discloses fungal cellulase produced from Humicola insolens. Suitable cellulases are also disclosed in GB-A-2.075.028 ; GB-A-2.095.275 and DE-OS-2.247.832.

Examples of such cellulases are cellulases produced by a strain of Humicola insulens (Humicola grisea var. thermoidea), particularly the Humicola strain DSM 1800, and cellulases produced by a fungus of Bacillus N or a cellulase 212-producing fungus belonging to the genus Aeromonas, and cellulase extracted from the hepatopancreas of a marine mollusc (Dolabella Auricula Solander).
Other suitable cellulases are cellulases originated from Humicola Insulens having a molecular weight of about 50KDa, an isoelectric point of 5.5 and containing 415 amino acids. Such cellulase are described in Copending European patent application No. 93200811.3, filed March 19, 1993.
Especially suitable cellulase are the cellulase having color care benefits. Examples of such cellulases are cellulase described in European patent application No. 91202879.2, filed November 6, 1991 Carezyme (Novo). If the detergent active compound is an enzyme, the silicone oil to enzyme-filled silica ratio should be at least 1.5:1.

Suitable detergent active compounds can also be represented by bleaches, bleach activator and bleach catalysts. Suitable inorganic bleaches include perborates, percarbonates. Suitable organic bleaches include peroxyacids known in art. Suitable bleach precursors are peracetic acid bleach precursors such as tetraacetylethylenediamine, triacetin, and acethyl trimethyl citrate.

Other detergent active compounds suitable for the present invention are fabric softening or conditioning agents, polymers, fluorescers, dyes, photoactivators through the wash sanitizers such as phenoxyethanol, and other detergent active compounds which are water-soluble or water-dispersible and which tend to be unstable upon storage, and mixtures thereof.

In case the detergent active does not spontaneously wet the surfaces of the hydrophobic material, the detergent active compound is mixed with a surfactant before being absorbed into the porous material.

Surfactants suitable for the present invention should be compatible with the detergent active compound. The surfactant to be used for instance, in the case the detergent active is an enzyme, a surfactant used is preferably a nonionic surfactant. A wide range of nonionic surfactants can be used.

One class of nonionic surfactants useful in the present invention are condensates of ethylene oxide with a hydrophobic moiety to provide a surfactant having an average hydrophilic-lipophilic balance (HLB) in the range from 8 to 17, preferably from 9.5 to 13.5, more preferably from 10 to 12.5. The hydrophobic (lipophilic) moiety may be aliphatic or aromatic in nature and the length of the polyoxyethylene group which is condensed with any particular hydrophobic group can be readily adjusted to yield a water-soluble compound having the desired degree of balance between hydrophilic and hydrophobic elements.

Especially preferred nonionic surfactants of this type are the C₉-C₁₅ primary alcohol ethoxylates containing 3-8 moles of ethylene oxide per mole of alcohol, particularly the C₁₄-C₁₅ primary alcohols containing 3-5 moles of ethylene oxide per mole of alcohol and the C₁₂-C₁₄ primary alcohols containing 3-5 moles of ethylene oxide per mole of alcohol.

A highly preferred class of nonionic surfactant to be used include poly hydroxy fatty acid amide surfactants of the formula 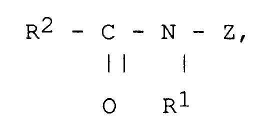
wherein R¹ is H, or R¹ is C₁₋₄ hydrocarbyl, 2-hydroxy ethyl, 2-hydroxy propyl or a mixture thereof, R² is C₅₋₃₁ hydrocarbyl, and Z is a polyhydroxyhydrocarbyl having a linear hydrocarbyl chain with at least 3 hydroxyls directly connected to the chain, or an alkoxylated derivative thereof. Preferably, R¹ is methyl, R² is a straight C₁₁₋₁₅ alkyl or alkenyl chain such as coconut alkyl or mixtures thereof, and Z is derived from a reducing sugar such as glucose, fructose, maltose, lactose, in a reductive amination reaction.

Anionic or cationic surfactants are less likely to be used. However, if the absorbed detergent active is unaffected by these surfactants they can alternatively be used.

The active-filled porous hydrophobic material may contain additional ingredients, which can be premixed with the surfactant before they are absorbed into the porous hydrophobic material.
The porous materials according to the present invention can include other detergent active compounds such as polymers, perfumes, brighteners, bleaches, softeners and other conventional optional ingredients such as buffers, electrolytes, etc. as far as they are chemically compatible with the surfactant active solution.

### Detergent ingredients

In another embodiment of the present invention, a liquid detergent composition is provided comprising the dye transfer inhibiting composition mixed with detergent ingredients. A wide range of surfactants can be used in the detergent composition of the present invention.

A typical listing of anionic, nonionic, ampholytic and zwitterionic classes, and species of these surfactants, is given in US Patent 3,664,961 issued to Norris on May 23, 1972.

Preferred anionic surfactants include the alkyl sulfate surfactants hereof are water soluble salts or acids of the formula ROSO₃M wherein R preferably is a C₁₀-C₂₄ hydrocarbyl, preferably an alkyl or hydroxyalkyl having a C₁₀-C₂₀ alkyl component, more preferably a C₁₂-C₁₈ alkyl or hydroxyalkyl, and M is H or a cation, e.g., an alkali metal cation (e.g. sodium, potassium, lithium), or ammonium or substituted ammonium (e.g. methyl-, dimethyl-, and trimethyl ammonium cations and quaternary ammonium cations such as tetramethyl-ammonium and dimethyl piperdinium cations and quaternary ammonium cations derived from alkylamines such as ethylamine, diethylamine, triethylamine, and mixtures thereof, and the like). Typically, alkyl chains of C₁₂-C₁₆ are preferred for lower wash temperatures (e.g. below about 50°C) and C₁₆₋₁₈ alkyl chains are preferred for higher wash temperatures (e.g. above about 50°C).

Highly preferred anionic surfactants include alkyl alkoxylated sulfate surfactants hereof are water soluble salts or acids of the formula RO(A)ₘSO3M wherein R is an unsubstituted C₁₀-C₂₄ alkyl or hydroxyalkyl group having a C₁₀-C₂₄ alkyl component, preferably a C₁₂-C₂₀ alkyl or hydroxyalkyl, more preferably C₁₂-C₁₈ alkyl or hydroxyalkyl, A is an ethoxy or propoxy unit, m is greater than zero, typically between about 0.5 and about 6, more preferably between about 0.5 and about 3, and M is H or a cation which can be, for example, a metal cation (e.g., sodium, potassium, lithium, calcium, magnesium, etc.), ammonium or substituted-ammonium cation. Alkyl ethoxylated sulfates as well as alkyl propoxylated sulfates are contemplated herein. Specific examples of substituted ammonium cations include methyl-, dimethyl, trimethyl-ammonium cations and quaternary ammonium cations such as tetramethyl-ammonium and dimethyl piperdinium cations and those derived from alkylamines such as ethylamine, diethylamine, triethylamine, mixtures thereof, and the like. Exemplary surfactants are C₁₂-C₁₈ alkyl polyethoxylate (1.0) sulfate (C₁₂-C₁₈E(1.0)M), C₁₂-C₁₈ alkyl polyethoxylate (2.25) sulfate (C₁₂-C₁₈E(2.25)M), C₁₂-C₁₈ alkyl polyethoxylate (3.0) sulfate (C₁₂-C₁₈E(3.0)M), and C₁₂-C₁₈ alkyl polyethoxylate (4.0) sulfate (C₁₂-C₁₈E(4.0)M), wherein M is conveniently selected from sodium and potassium.

Other suitable anionic surfactants to be used are alkyl ester sulfonate surfactants including linear esters of C₈-C₂₀ carboxylic acids (i.e., fatty acids) which are sulfonated with gaseous SO₃ according to "The Journal of the American Oil Chemists Society", 52 (1975), pp. 323-329. Suitable starting materials would include natural fatty substances as derived from tallow, palm oil, etc.

The preferred alkyl ester sulfonate surfactant, especially for laundry applications, comprise alkyl ester sulfonate surfactants of the structural formula : 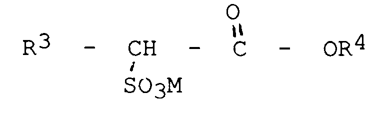
wherein R³ is a C₈-C₂₀ hydrocarbyl, preferably an alkyl, or combination thereof, R⁴ is a C₁-C₆ hydrocarbyl, preferably an alkyl, or combination thereof, and M is a cation which forms a water soluble salt with the alkyl ester sulfonate. Suitable salt-forming cations include metals such as sodium, potassium, and lithium, and substituted or unsubstituted ammonium cations, such as monoethanolamine, diethanolamine, and triethanolamine. Preferably, R³ is C₁₀-C₁₆ alkyl, and R⁴ is methyl, ethyl or isopropyl. Especially preferred are the methyl ester sulfonates wherein R³ is C₁₀-C₁₆ alkyl.

Other anionic surfactants useful for detersive purposes can also be included in the laundry detergent compositions of the present invention. These can include salts (including, for example, sodium, potassium, ammonium, and substituted ammonium salts such as mono-, di- and triethanolamine salts) of soap, C₉-C₂₀ linear alkylbenzenesulfonates, C₈-C₂₂ primary of secondary alkanesulfonates, C₈-C₂₄ olefinsulfonates, sulfonated polycarboxylic acids prepared by sulfonation of the pyrolyzed product of alkaline earth metal citrates, e.g., as described in British patent specification No. 1,082,179, C₈-C₂₄ alkylpolyglycolethersulfates (containing up to 10 moles of ethylene oxide); alkyl glycerol sulfonates, fatty acyl glycerol sulfonates, fatty oleyl glycerol sulfates, alkyl phenol ethylene oxide ether sulfates, paraffin sulfonates, alkyl phosphates, isethionates such as the acyl isethionates, N-acyl taurates, alkyl succinamates and sulfosuccinates, monoesters of sulfosuccinates (especially saturated and unsaturated C₁₂-C₁₈ monoesters) and diesters of sulfosuccinates (especially saturated and unsaturated C₆-C₁₂ diesters), acyl sarcosinates, sulfates of alkylpolysaccharides such as the sulfates of alkylpolyglucoside (the nonionic nonsulfated compounds being described below), branched primary alkyl sulfates, and alkyl polyethoxy carboxylates such as those of the formula RO(CH₂CH₂O)ₖ-CH₂COO-M+ wherein R is a C₈-C₂₂ alkyl, k is an integer from 0 to 10, and M is a soluble salt-forming cation. Resin acids and hydrogenated resin acids are also suitable, such as rosin, hydrogenated rosin, and resin acids and hydrogenated resin acids present in or derived from tall oil. Further examples are described in "Surface Active Agents and Detergents" (Vol. I and II by Schwartz, Perry and Berch). A variety of such surfactants are also generally disclosed in U.S. Patent 3,929,678, issued December 30, 1975 to Laughlin, et al. at Column 23, line 58 through Column 29, line 23 (herein incorporated by reference).

When included therein, the laundry detergent compositions of the present invention typically comprise from about 1% to about 40%, preferably from about 3% to about 20% by weight of such anionic surfactants.

One class of nonionic surfactants useful in the present invention are condensates of ethylene oxide with a hydrophobic moiety to provide a surfactant having an average hydrophiliclipophilic balance (HLB) in the range from 8 to 17, preferably from 9.5 to 14, more preferably from 12 to 14. The hydrophobic (lipophilic) moiety may be aliphatic or aromatic in nature and the length of the polyoxyethylene group which is condensed with any particular hydrophobic group can be readily adjusted to yield a water-soluble compound having the desired degree of balance between hydrophilic and hydrophobic elements. In case the liquid detergent is structured with clay, the HLB value should preferably be less than 10.

Especially preferred nonionic surfactants of this type are the C₉-C₁₅ primary alcohol ethoxylates containing 3-12 moles of ethylene oxide per mole of alcohol, particularly the C₁₂-C₁₅ primary alcohols containing 5-8 moles of ethylene oxide per mole of alcohol.

Another class of nonionic surfactants comprises alkyl polyglucoside compounds of general formula

RO (CₙH₂ₙO)ₜZₓ

wherein Z is a moiety derived from glucose; R is a saturated hydrophobic alkyl group that contains from 12 to 18 carbon atoms; t is from 0 to 10 and n is 2 or 3; x is from 1.3 to 4, the compounds including less than 10% unreacted fatty alcohol and less than 50% short chain alkyl polyglucosides. Compounds of this type and their use in detergent are disclosed in EP-B 0 070 077, 0 075 996 and 0 094 118.

Very suitable as nonionic surfactants are poly hydroxy fatty acid amide surfactants of the formula 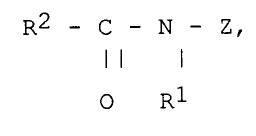
wherein R¹ is H, or R¹ is C₁₋₄ hydrocarbyl, 2-hydroxy ethyl, 2-hydroxy propyl or a mixture thereof, R² is C₅₋₃₁ hydrocarbyl, and Z is a polyhydroxyhydrocarbyl having a linear hydrocarbyl chain with at least 3 hydroxyls directly connected to the chain, or an alkoxylated derivative thereof. Preferably, R¹ is methyl, R² is a straight C₁₁₋₁₅ alkyl or alkenyl chain such as coconut alkyl or mixtures thereof, and Z is derived from a reducing sugar such as glucose, fructose, maltose, lactose, in a reductive amination reaction.

Cationic detersive surfactants suitable for use in the laundry detergent compositions of the present invention are those having one long-chain hydrocarbyl group. Examples of such cationic surfactants include the ammonium surfactants such as alkyldimethylammonium halogenides, and those surfactants having the formula :

[R²(OR³)_{y}][R⁴(OR³)_{y}]₂R⁵N+X-

wherein R² is an alkyl or alkyl benzyl group having from about 8 to about 18 carbon atoms in the alkyl chain, each R³ is selected from the group consisting of -CH₂CH₂-, -CH₂CH(CH₃)-, -CH₂CH(CH₂OH)-, -CH₂CH₂CH₂-, and mixtures thereof; each R⁴ is selected from the group consisting of C₁-C₄ alkyl, C₁-C₄ hydroxyalkyl, benzyl ring structures formed by joining the two R⁴ groups, -CH₂CHOH-CHOHCOR⁶CHOHCH₂OH wherein R⁶ is any hexose or hexose polymer having a molecular weight less than about 1000, and hydrogen when y is not 0; R⁵ is the same as R⁴ or is an alkyl chain wherein the total number of carbon atoms of R² plus R⁵ is not more than about 18; each y is from 0 to about 10 and the sum of the y values is from 0 to about 15; and X is any compatible anion.

Preferred cationic surfactants are the water-soluble quaternary ammonium compounds useful in the present composition having the formula :

R₁R₂R₃R₄N⁺X⁻ (i)

wherein R₁ is C₈-C₁₆ alkyl, each of R₂, R₃ and R₄ is independently C₁-C₄ alkyl, C₁-C₄ hydroxy alkyl, benzyl, and - (C₂H₄0)ₓH where x has a value from 1 to 5, and X is an anion. Not more than one of R₂, R₃ or R₄ should be benzyl.
The preferred alkyl chain length for R₁ is C₁₂-C₁₅ particularly where the alkyl group is a mixture of chain lengths derived from coconut or palm kernel fat or is derived synthetically by olefin build up or OXO alcohols synthesis. Preferred groups for R₂R₃ and R₄ are methyl and hydroxyethyl groups and the anion X may be selected from halide, methosulphate, acetate and phosphate ions.

Examples of suitable quaternary ammonium compounds of formulae (i) for use herein are :
coconut trimethyl ammonium chloride or bromide;
coconut methyl dihydroxyethyl ammonium chloride or bromide;
decyl triethyl ammonium chloride;
decyl dimethyl hydroxyethyl ammonium chloride or bromide;
C₁₂₋₁₅ dimethyl hydroxyethyl ammonium chloride or bromide;
coconut dimethyl hydroxyethyl ammonium chloride or bromide;
myristyl trimethyl ammonium methyl sulphate;
lauryl dimethyl benzyl ammonium chloride or bromide;
lauryl dimethyl (ethenoxy)₄ ammonium chloride or bromide;
choline esters (compounds of formula (i) wherein R₁ is 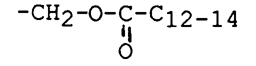
alkyl and R₂R₃R₄ are methyl).

di-alkyl imidazolines [compounds of formula (i)].

Other cationic surfactants useful herein are also described in U.S. Patent 4,228,044, Cambre, issued October 14, 1980.

When included therein, the laundry detergent compositions of the present invention typically comprise from 0.5% to about 5%, preferably from about 1% to about 3% by weight of such cationic surfactants.

The compositions according to the present invention may further comprise a builder system. Any conventional builder system is suitable for use herein including aluminosilicate materials, silicates, polycarboxylates and fatty acids, materials such as ethylenediamine tetraacetate, metal ion sequestrants such as aminopolyphosphonates, particularly ethylenediamine tetramethylene phosphonic acid and diethylene triamine pentamethylenephosphonic acid. Though less preferred for obvious environmental reasons, phosphate builders can also be used herein.
Suitable polycarboxylates builders for use herein include citric acid, preferably in the form of a water-soluble salt, derivatives of succinic acid of the formula R-CH(COOH)CH2(COOH) wherein R is C10-20 alkyl or alkenyl, preferably C12-16, or wherein R can be substituted with hydroxyl, sulfo sulfoxyl or sulfone substituents. Specific examples include lauryl succinate , myristyl succinate, palmityl succinate2-dodecenylsuccinate, 2-tetradecenyl succinate. Succinate builders are preferably used in the form of their water-soluble salts, including sodium, potassium, ammonium and alkanolammonium salts.
Other suitable polycarboxylates are oxodisuccinates and mixtures of tartrate monosuccinic and tartrate disuccinic acid such as described in US 4,663,071.
Especially for the liquid execution herein, suitable fatty acid builders for use herein are saturated or unsaturated C10-18 fatty acids, as well as the corresponding soaps. Preferred saturated species have from 12 to 16 carbon atoms in the alkyl chain. The preferred unsaturated fatty acid is oleic acid. Another preferred builder system for liquid compositions is based on dodecenyl succinic acid.

Detergency builder salts are normally included in amounts of from 3% to 50% by weight of the composition preferably from 5% to 30% and most usually from 5% to 25% by weight.

Other components used in detergent compositions may be employed, such as enzymes and stabilizers or activators therefore, soil-suspending agents, soil-release polymers, other optical brighteners, abrasives, bactericides, tarnish inhibitors, coloring agents, foam control agents, corrosion inhibitors and perfumes. Especially preferred are combinations with enzyme technologies which also provide a type of color care benefit. Examples are cellulase for color maintenance/rejuvenation. Other examples are the polymers disclosed in EP 92870017.8 filed January 31,1992 and enzyme oxidation scavengers disclosed in EP 92870018.6 filed January 31, 1992.
Also particulary suitable are amine base catalyst stabilizers disclosed in EP 92870019.4 filed January 31, 1992.

Preferably the liquid compositions according to the present invention are in "concentrated form"; in such case, the liquid detergent compositions according to the present invention will contain a lower amount of water, compared to conventional liquid detergents. The level of water is less than 50%, preferably less than 40%, more preferably less than 30% of water by weight of the detergent compositions.
Said concentrated products provide advantages to the consumer, who has a product which can be used in lower amounts and to the producer, who has lower shipping costs.

The detergent compositions according to the present invention include compositions which are to be used for cleaning substrates, such as fabrics, fibers, hard surfaces, skin etc., for example hard surface cleaning compositions (with or without abrasives), laundry detergent compositions, automatic and non automatic dishwashing compositions.

The following examples are meant to exemplify compositions of the present inventions, but are not necessarily meant to limit the scope of the invention.

### Test procedure

The stability of the structured liquid detergent compositions containing the porous hydrophobic material according to the present invention was measured upon storage at room temperature. More in particular, the structured liquid detergent compositions of Table I were supplemented with caged cellulase (Carezyme) as indicated below :

| **Silica/cellulase** | | | | |
|---|---|---|---|---|
| Silica | Cellulase solution***** | ratio sil./cell. solution | extra addition | sil.oil 200 fluid******* |
| Sipernat D10 | | 1:2.5 | 5% Aerosil of R972****** | 1000 cs |

| | | | | |
|---|---|---|---|---|
| ***** The cellulase solution contains 4.9% polyhydroxy amide, 95% cellulase raw material solution and 0.1% polyethylene glycol. | | | | |
| ****** Aerosil 5% based on the total weight of the cellulase filled silica. | | | | |
| ******* The weight ratio of silicone oil to cellulase filled silica was 1.8. | | | | |

The following liquid detergent compositions were made :

**Table I**

| | **I** | **II** | **III** | **IV** | **V** |
|---|---|---|---|---|---|
| Linear alkylbenzene sulfonate | 18 | - | - | 10 | - |
| C₁₂-C₁₅ Alkyl sulfate | 12 | 12.4 | 18 | - | 10 |
| C₁₂-C₁₅ Alkyl ethoxylated sulfate | 3 | 2.4 | 5 | - | 5 |
| C₁₂-C₁₄ N-methyl glucamide | 4 | 4 | 6 | 6 | 6 |
| Coconut dimethyl hydroxy ethyl ethyl ammonium chlorides | 2 | 2 | 2 | - | 2 |
| C₁₂-C₁₆ Fatty acid | 17 | 16.5 | 10 | 10 | 10 |
| Citric acid anhydrous | 1.5 | 1.5 | 5 | - | 1.5 |
| Smectite clay | 3.5 | 3.4 | 3.5 | - | - |
| Hectorite organoclay | 0.5 | 0.4 | 0.5 | - | - |
| NaOH | 6.5 | 4.7 | 5 | - | - |
| Propanediol | 8.5 | 9.0 | 9 | 9 | 9 |
| Ethanol | 1.5 | 1.0 | 1 | 1 | 1 |
| FNA-Base (34g/l) | 0.7 | 0.72 | 0.7 | 0.7 | 0.7 |
| Termamyl (300KNU/g) | 0.3 | 0.26 | 0.2 | 0.2 | 0.2 |
| Lipolase | - | 0.2 | 0.2 | 0.2 | 0.2 |
| Polyethylene oxide | - | - | 0.2 | - | - |
| Boric acid | 1.2 | 1.2 | 2 | 2 | 2 |
| Silane | 0.03 | 0.03 | 0.03 | 0.03 | 0.03 |
| Silicone | 0.25 | 0.2 | 0.25 | 0.25 | 0.25 |
| Dispersant | 0.05 | 0.05 | 0.05 | 0.05 | 0.05 |
| Water | 20 | 40 | 30 | 60 | 50 |
| Minors | ------up to 100%------ | | | | |

Structured liquid detergent compositions according to the present invention (I, II, III, IV and V) are stable, even after prolonged periods of storage.

## Claims

1. A liquid detergent composition containing a porous hydrophobic material, the porous hydrophobic material having a detergent active absorbed therein, said porous material being coated with a hydrophobic material, characterized in that said liquid detergent composition is a structured liquid detergent composition.

2. A liquid detergent composition according to claim 1, wherein said detergent active compound is selected from enzymes, bleaches, bleach activators. bleach catalysts, perfumes, photoactivators, dyes, brighteners/fluorescers, through the wash sanitizers, fabric softening or conditioning agents, hydrolysable surfactants, polymers and other detergent active compounds which are water-soluble or water-dispersible and mixtures thereof.

3. A liquid detergent composition according to claims 1-2, wherein said liquid detergent composition is externally structured.

4. A liquid detergent composition according to claim 3, being structured by clay.

5. A liquid detergent composition according to claim 1-2 wherein said liquid detergent composition is internally structured.

6. A liquid detergent composition according to claim 5, being structured by polyhydroxy amide surfactant.

7. A liquid detergent composition according to claims 1-6 wherein said porous hydrophobic is silica and wherein said hydrophobic coating material is silicone oil.

8. Use of the liquid detergent composition according to claim 1-7 for cleaning of fabrics, fibers, hard surfaces and skin.
